# EUROPÄISCHE PATENTSCHRIFT

(11) **EP 2 288 701 B1**
(45) Veröffentlichungstag und Bekanntmachung des Hinweises auf die Patenterteilung: **17.06.2015**
(21) Anmeldenummer: 09765879.3
(22) Anmeldetag: 18.06.2009
(51) Int. Cl.: C12N 15/10

(54) **VERFAHREN ZUR AUFREINIGUNG VON NUKLEINSÄUREN, INSBESONDERE AUS FIXIERTEM GEWEBE**
METHOD FOR FILTERING NUCLEIC ACIDS, IN PARTICULAR FROM FIXED TISSUE
PROCÉDÉ DE PURIFICATION D'ACIDES NUCLÉIQUES, PROVENANT EN PARTICULIER DE TISSUS FIXÉS

(30) Priorität: 20.06.2008 DE 102008029356
(43) Veröffentlichungstag der Anmeldung: 02.03.2011
(73) Patentinhaber: Siemens Healthcare Diagnostics GmbH, 65760 Eschborn (DE)
(72) Erfinder: EUTING, Heike, 46399 Bocholt (DE); HENNIG, Guido, 50859 Köln (DE); BOHMANN, Kerstin, 50677 Köln (DE)
(74) Vertreter: Schweitzer, Klaus
(86) Internationale Anmeldenummer: PCT/EP2009/057561
(87) Internationale Veröffentlichungsnummer: WO 2009/153299

(56) Entgegenhaltungen:
- EP-A2- 0 389 063
- WO-A1-01/46402
- WO-A1-03/040364
- WO-A1-03/058649
- PETRY C ET AL: "[Predictive and prognostic markers of breast cancer. Molecular biological analysis of fixed tumor tissue]" DER PATHOLOGE NOV 2008, Bd. 29 Suppl 2, November 2008 (2008-11), Seiten 181-183, XP002547507 ISSN: 1432-1963
- GILBERT M THOMAS P ET AL: "The isolation of nucleic acids from fixed, paraffin-embedded tissues-which methods are useful when?" PLOS ONE, Bd. 2, Nr. 6, 2007, Seiten E537/1-E537/12, XP002547508 ISSN: 1932-6203
- SATO Y ET AL: "COMPARISON OF THE DNA EXTRACTION METHODS FOR POLYMERASE CHAIN REACTION AMPLIFICATION FROM FORMALIN-FIXED AND PARAFFIN-EMBEDDED TISSUES" DIAGNOSTIC MOLECULAR PATHOLOGY, Bd. 10, Nr. 4, 1. Dezember 2001 (2001-12-01), Seiten 265-271, XP009010319 NEW YORK, NY, US
- WATSON R M ET AL: "Increased sample capacity for genotyping and expression profiling by kinetic polymerase chain reaction" ANALYTICAL BIOCHEMISTRY, Bd. 329, Nr. 1, 1. Juni 2004 (2004-06-01), Seiten 58-67, XP004506465 ACADEMIC PRESS INC, NEW YORK ISSN: 0003-2697
- HOFMANN W P ET AL: "Comparison of transcription mediated amplification (TMA) and reverse transcription polymerase chain reaction (RT-PCR) for detection of hepatitis C virus RNA in liver tissue" JOURNAL OF CLINICAL VIROLOGY, Bd. 32, Nr. 4, 1. April 2005 (2005-04-01), Seiten 289-293, XP004797283 ELSEVIER, AMSTERDAM, NL ISSN: 1386-6532
- BOHMANN KERSTIN ET AL: "RNA extraction from archival formalin-fixed paraffin-embedded tissue: a comparison of manual, semiautomated, and fully automated purification methods", CLINICAL CHEMISTRY, AMERICAN ASSOCIATION FOR CLINICAL CHEMISTRY, WASHINGTON, DC, vol. 55, no. 9, 1 September 2009 (2009-09-01), pages 1719-1727, XP009130062, ISSN: 0009-9147 & MÜLLER BERIT MARIA ET AL: "Quantitative determination of estrogen receptor, progesterone receptor, and HER2 mRNA in formalin-fixed paraffin-embedded tissue--a new option for predictive biomarker assessment in breast cancer.", DIAGNOSTIC MOLECULAR PATHOLOGY : THE AMERICAN JOURNAL OF SURGICAL PATHOLOGY, PART B MAR 2011, vol. 20, no. 1, March 2011 (2011-03), pages 1-10, XP009169635, ISSN: 1533-4066

## Beschreibung

Die Erfindung betrifft ein Verfahren zur Aufreinigung von Nukleinsäuren, ein Kit, um das erfindungsgemäße Verfahren durchzuführen, sowie eine neue Verwendung von Magnetpartikeln zur Aufreinigung einer biologischen Probe.

In jüngster Zeit gewinnt die Molekulare Diagnostik zunehmend an Bedeutung. Sie hat Eingang gefunden in die klinische Diagnostik von Erkrankungen (u. a. Nachweis von Infektionserregern, Nachweis von Mutationen des Genoms, Entdeckung von zirkulierenden Tumorzellen und Identifizierung von Risikofaktoren für die Prädisposition einer Erkrankung). Aber auch in der Veterinärmedizin, der Umweltanalytik und Nahrungsmitteltestung finden Methoden der molekularen Diagnostik mittlerweile ihre Anwendung. Ein weiteres Anwendungsgebiet stellen Untersuchungen an pathologischen/zytologischen Instituten oder im Rahmen forensischer Fragestellungen dar. Aber auch im Rahmen der Gesundheitsvorsorge (z.B. Untersuchungen von Blutkonserven auf Infektionserreger-Freiheit) wird die Gen-Diagnostik mittlerweile eingesetzt und der Gesetzgeber plant, solche Tests per Gesetz in Zukunft anzuordnen. Methoden, die auch bei der klinischen Molekularen Diagnostik zum Einsatz kommen (wie z.B. Hybridisierungs- oder Amplifikationstechniken wie die PCR (Polymerase Chain Reaction), TMA (Transcription mediated amplification), LCR (Ligase Chain Reaction), bDNA (branched DNA) oder NASBA (Nucleic Acid Sequence Based Amplification)-Technologie) gehören auch bei wissenschaftlichen Grundlagenarbeiten zu den Routineverfahren.

Insbesondere eröffnet die Nukleinsäureanalytik durch die Bestimmung der Genexpression in Geweben vielversprechende neue Möglichkeiten in der Erforschung und Diagnostik von Tumorerkrankungen. So haben beispielsweise die sogenannten Mikroarray-Systeme die Möglichkeit eröffnet, in einem einzigen Ansatz die Expression von hunderten oder sogar von tausenden von Genen zu bestimmen. Dabei wird das Probenmaterial, aufgereinigte Nukleinsäuren, z.B. RNA oder cDNA, auf einen Chip aufgebracht, welcher entsprechende Fänger-Oligonukleotide aufweist, so dass die Nukleinsäuren in der Probe durch Hybridisieren nachgewiesen werden können. Daneben existieren auch weit verbreitet andere Verfahren zum Nachweis von Nukleinsäuren in einer Probe, z.B. Amplifikationsverfahren wie die Polymerase Kettenreaktion (PCR).

Ein grundsätzliches Problem in der Nukleinsäureanalytik ist die Probenaufbereitung. Die zu untersuchende Probe enthält meist Zellen oder Gewebe mit störenden, teilweise unlöslichen Bestandteilen (sog. Debris), welche die nachfolgende Isolierung und Analyse stören können. Insbesondere bei der Nukleinsäureisolation von Stuhl/Faeces, Blut, Warzen, verkalkten Strukturen (Knochen) oder auch stark nekrotischem Gewebeproben treten solche unlösliche Bestandteile auf. Debris kann im weitesten Sinne aber auch lösliche Komponenten beinhalten, z.B. freigesetztes Hämoglobin aus Erythrozyten, welches in extremem Überschuss vorhanden ist und während der Isolation von den Nukleinsäuren abgetrennt werden soll.

Dieses Problem ist insbesondere in der Tumordiagnostik gravierend, weil hier als Probenmaterial häufig formalinfixierte Paraffinschnitte (formalin-fixed paraffin-embedded, FFPE-Schnitte) verwendet werden. Bei der Probenentnahme am Patienten, z.B. bei Biopsieentnahme oder intraoperativer Probenentnahme von Tumormaterial, wird Gewebematerial mit Formalin fixiert und in Paraffin eingebettet, um das Probenmaterial haltbar zu machen. Durch die Fixantien kommt es während der Inkubation - aber auch noch über Jahre hinaus innerhalb des Gewebeblockes - zu einer extremen Kreuzvernetzung von Biomolekülen (Nukleinsäuren mit Proteinen sowie jeweils Proteine oder Nukleinsäuren untereinander). Diese vernetzten Strukturen innerhalb und außerhalb von Zellen tragen zur Entstehung von unlöslichem bzw. nicht oder schwer lysierbarem Debris bei. Von den im Paraffin eingebetteten Proben werden üblicherweise Schnitte zur Begutachtung durch Pathologen angefertigt, diese Schnitte können jedoch auch als Ausgangsmaterial in der Nukleinsäureanalytik dienen. Dabei muss bei der Aufreinigung der Nukleinsäuren nach der Lyse sowohl zelluläres Debris als auch das Paraffin entfernt werden.

Darüber hinaus tritt dieses Problem mit störenden, teilweise unlöslichen Bestandteilen (Debris) auch bei der Aufreinigung von Nukleinsäuren aus Stuhlproben (Faeces, Kot) auf. Stuhlproben bestehen zum einen aus den unverdaulichen Anteilen der Nahrung (Ballaststoffe) sowie unverdauten Resten wie Fett, Stärke, Bindegewebs- und Muskelfasern und Wasser, die nicht in den oberen Dickdarmabschnitten resorbiert wurden. An körpereigenen Substanzen sind enthalten: abgestoßene Darmzellen, Rückstände von Verdauungsenzymen und Schleim. Des Weiteren werden geringe Mengen der Gallensäuren selbst, sowie des zum Schutz der Darmschleimhaut ebenfalls von der Galle ausgeschiedenen Lecithins und anderer Phospholipide zusammen mit dem Kot ausgeschieden.

Um Kosten zu senken und die Bearbeitungszeit vom Probeneingang bis zur Bestimmung des Analyseergebnisses so kurz wie möglich zu halten, ist es ein vordringliches Ziel, Verfahren zur Aufreinigung von Nukleinsäuren so effizient wie möglich zu machen und soweit wie möglich automatisiert durchzuführen. Dies gilt insbesondere in der Diagnostik. Gut geeignet zur Automatisierung sind solche Verfahren, welche in möglichst wenig verschiedenen Reaktionsgefäßen durchgeführt werden können und in standardisierten Formaten (z.B. 96-Loch Plattenformat) durchgeführt werden können, weil bei diesem Verfahren effiziente Pipetierroboter eingesetzt werden können. Daher besteht im Stand der Technik das Bedürfnis nach einer einfachen, effizienten und möglichst automatisierbaren Probenaufbereitung.

Übliche Verfahren zur Nukleinsäureaufreinigung umfassen die Probenlyse unter chaotropen Bedingungen, Aufreinigung durch Extraktion, Ausfällen und Aufreinigen aus der flüssigen Phase, z.B. die sog. Phenol-Chloroform-Extraktion (siehe Sambrook et al., Molecular cloning - a laboratory manual, 3. Auflage, Cold Spring Harbor Laboratory, Cold Spring Habor Press, 2001, ISBN-13: 978-0879695774), oder sogenannte säulenbasierte Aufreinigungsverfahren wie beispielsweise offenbart in WO 2003040364-A1.

Ein übliches Verfahren zur Isolierung von Nukleinsäuren wurde von Chomczynski beschrieben (US 5,346,994) und umfasst die Aufreinigung von Nukleinsäuren aus Gewebematerial basierend auf einer Trennung aus der flüssigen Phase unter Verwendung von Phenol und der chaotropen Verbindung Guanidinisothiocyanat. Dabei muss die Probe in wässriger Lösung homogenisiert und nach Zugabe von Guanidinisothiocyanat (GTC) und Phenol/ Chloroform zentrifugiert werden. Proteine befinden sich in der organischen Phase, DNA in der Interphase und RNA in der wässrigen Phase. Die RNA kann aus der wässrigen Phase präzipitiert werden. Dieses Verfahren ermöglicht jedoch nicht die zuverlässige Aufreinigung von RNA aus FFPE-Gewebeproben.

Andere bekannte Verfahren zur DNA- oder RNA-Isolierung verwenden typischer Weise chaotrope Salze oder Phenolextraktion.

EP0819696 offenbart ein Verfahren zur Aufreinigung von Nukleinsäuren, das auf der Bindung von Nukleinsäuren an Silika oder andere Siliziumdioxidderivate unter chaotropen Bedingungen basiert. Dabei wird die Probe in einem chaotropen Lysepuffer lysiert und die Nukleinsäuren an eine Silikamatrix gebunden.

Im Stand der Technik bekannte Verfahren zur Aufreinigung von Nukleinsäuren aus Paraffinschnitten erfordern zunächst eine aufwändige Entparaffinisierung, wobei das Paraffin typischerweise durch Xylol entfernt wird, sowie eine aufwändige anschließende Rehydratisierung mit einer Xylol/Ethanol Verdünnungsreihe.

So wird in der WO 200146402 A1 ein Verfahren zur Aufreinigung von RNA aus fixierten Paraffinschnitten beschrieben, bei welchem der Paraffinschnitt zunächst in ein Eppendorf-Reaktionsgefäß eingebracht und mit Xylol entparaffinisiert wird. Anschließend muss der Schnitt mit einer Xylol/Ethanol Verdünnungsreihe rehydratisiert werden. Anschließend wird zur Aufreinigung der RNA die Probe über einen längeren Zeitraum (5 bis 120 min.) in einer chaotropen Lösung erhitzt. Dieses Verfahren ermöglicht zwar eine effektive Entparaffinisierung, ist aber aufwändig und aufgrund mehrerer erforderlicher Zentrifugationsschritte für eine Automatisierung nicht sehr geeignet.

In WO03/058649-A1 werden Nukleinsäuren aus Serum, aber auch aus Paraffinschnitten gereinigt, indem die Proben nach Lyse in einem chaotropen Lyse- und Bindungspuffer mit SiO₂-umhüllten Magnetitpartikeln der Sorte Bayoxide E8707 vermischt werden. Die in der Probe befindliche DNA bzw. RNA bindet an diese Partikel. Beim Entfernen des Überstands und den nachfolgenden Waschschritten werden die Bayoxide E8707-Partikel durch ein Magnetfeld im Probengefäss zurückgehalten. Abschliessend werden die Nukleinsäuren von den magnetischen Partikeln eluiert. Bei dieser Methode findet vor der Zugabe des chaotropen Lyse- und Bindungspuffers keine Abtrennung der Debris statt.

Gegenüber dem Stand der Technik besteht ein Bedürfnis nach verbesserten Verfahren zur Aufreinigung von Nukleinsäuren, und insbesondere für Verfahren, welche für eine Automatisierung geeignet sind.

### Definitionen

Der Ausdruck "biologische Probe" bezeichnet jegliche Probe, welche Zellen oder zelluläres Material enthält, insbesondere Zellen, gefrorene Zellpellets, fixierte Zellen, Faeces/Stuhl, "Buffy Coat" (= weiße Blutkörperchenfraktion des Blutes), Ascites, Abstriche, insbesondere Backen- oder Rachenabstriche, ganz besonders aber Gebärmutterhalsabstriche, Sputum, Organpunktate, Sperma, Gewebeproben, fixierte Gewebeproben, Gewebeschnitte von fixierten oder nicht-fixierten Gewebeproben, insbesondere Gefrierschnitte und Paraffinschnitte, insbesondere formalinfixierte Paraffinschnitte, Tumormaterial, Biopsieproben, Blutproben, insbesondere Gesamtblut oder Blutfraktionen, Zellsuspensionen und im weitesten Sinne sämtliche Proben, welche zelluläre Bestandteile aufweisen, wobei sowohl intakte Zellen als auch Zellbestandteile umfasst sein sollen.

Darüber hinaus umfasst der Ausdruck "biologische Probe" auch andere nukleinsäurehaltigen, biologischen Materialen, wie z.B. Blutserum oder Blutplasma, insbesondere virushaltiges Serum oder Plasma, dabei ganz besonders HIV und HCV infizierte Serumproben, Sekrete, Liquor, Galle, Lymphflüssigkeit, Urin. Ebenso kann es sich um nukleinsäurehaltige Materialien handeln, welche aus biochemischen oder biotechnologischen Prozessen stammen und anschließend aufgereinigt werden sollen.

Der Begriff "zellulär" bezieht sich sowohl auf prokaryotische als auch eukaryotische Zellen.

Der Begriff "Lysieren der Probe" umfasst das Aufbrechen von Zellen oder zellulären Strukturen in der Probe. Er umfasst insbesondere mechanische Lyseverfahren (z.B. Ultraschall), thermische Lyse (z.B. Einfrier-Auftau-Zyklen, erhitzen der Probe) und chemische Lyse (z.B. mit Detergentien). Der Ausdruck "Lysieren der Probe" ist aber nicht auf Zellen beschränkt und kann sich auch auf das Freisetzen von Nukleinsäuren mit den beschriebenen Verfahren aus nicht-zellulären, biologischen Strukturen oder Komplexen beziehen.

Der Ausdruck "Nukleinsäuren" umfasst oligomere und polymere Ribonukleotide bzw. 2'-Desoxyribonukleotide mit einer Kettenlänge von mehr als 10 Monomereinheiten. Die Monomereinheiten in Nukleinsäuren sind über Phosphorsäurediesterbindungen zwischen 3'- und 5'-Hydroxylgruppe benachbarter Monomereinheiten verknüpft und an das 1'-Atom der jeweiligen Kohlenhydratkomponente ist glykosidisch eine heterocyclische Base gebunden. Nukleinsäuren können durch Ausbildung von intermolekularen Wasserstoffbrückenbindungen Doppel- und Dreifachstränge ausbilden.

Ebenso sind Protein-/Nukleinsäurekomplexe sowie Nukleinsäuren mit synthetischen Nukleotiden, wie Morpholinos, LNAs oder PNAs, gemeint.

Der Begriff "chaotrope Bedingungen" bezeichnet Lösungsmittelbedingungen bei Anwesenheit von chaotropen Agentien oder Verbindungen. Chaotrope Agentien oder Verbindungen sind Verbindungen, welche die Sekundärstruktur, Tertiärstruktur und Quaternärstruktur von Proteinen, Nukleinsäuren und Protein-Nukleinsäurekomplexen verändert oder aufbricht, während die Primärstruktur intakt bleibt. In Lösung werden unter chaotropen Bedingungen die intramolekularen Wechselwirkungen von biologischen Molekülen, insbesondere Proteinen, Protein-Nukleinsäurekomplexen und Nukleinsäuren aufgebrochen, da chaotrope Verbindungen mit stabilisierenden intramolekularen Wechselwirkungen in biologischen Molekülen, z.B. Wasserstoffbrückenbindungen, van der Wals-Kräfte und hydrophobe Effekte, interferieren. Chaotrope Verbindungen weisen üblicherweise großvolumige Ionen auf, die aufgrund Ihrer Größe mit den intramolekularen Wechselwirkungen interferieren und dadurch die Polarität des Lösungsmittels herabsetzen können. Dadurch werden inter- und intramoleklare Wasserstoffbrücken aufgebrochen. Infolgedessen präzipitieren viele Proteine, die Helixstruktur von zweisträngigen Nukleinsäureabschnitten bleibt jedoch erhalten. Durch Hinzufügen von chaotropen Verbindungen zu Zelllysaten oder Zellsuspensionen lassen sich Proteine ausfällen, während Nukleinsäuren in Lösung bleiben. Unter chaotropen Bedingungen wird die Bindung von Nukleinsäuren an Silizumdioxid-basierte Matrices stark begünstigt. Chaotrope Verbindungen umfassen beispielsweise hochmolekulare Harnstofflösungen (z.B. 6 bis 8 mol/l Harnstoff), Guanidiniumsalzlösungen (z.B. 6 mol/l Guanidiniumchlorid), hochmolekulare Lithiumsalze (z.B. 4,5 mol/l Lithiumperchlorat). Chaotrope Anionen umfassen die Anionen F-, P0₄³⁻, SO₄²⁻, CH₃C00⁻, Cl⁻, und insbesondere Br⁻, I⁻, NO₃⁻, Cl0₄⁻, SCN⁻ und Cl₃CCOO⁻. Chaotrope Kationen umfassen die Kationen Li⁺, Mg²⁺, Ca²⁺, Ba²⁺, und insbesondere das Guanidiniumkation, [CH₆N₃]⁺. Zur Nukleinsäureisolierung bevorzugte chaotrope Verbindung sind Guanidiniumisothiocyanat ([CH₆N₃]⁺ SCN⁻) und Guanidiniumchlorid.

Der Begriff "nicht-chaotrope Bedingungen" bezeichnet Lösungsmittelbedingungen in wässriger und/oder alkoholischer Lösung bei Abwesenheit von chaotropen Agenzien.

Der Begriff "Aufreinigen von Nukleinsäuren" beschreibt das unvollständige oder vollständige Entfernen von Nicht-Nukleinsäurebestandteilen aus einer Nukleinsäure enthaltenden Probe. Er ist nicht beschränkt auf das Erreichen eines bestimmten Reinheitsgrades.

Der Begriff "automatisierte Aufreinigung" umfasst Verfahren, die die manuelle Arbeitskraft von menschlichem Personal ganz oder auch nur in Teilschritten ersetzt und insbesondere bei den Schritten der Aufschließung der biologischen Körperprobe mit einem speziellen Puffer, der Zugabe von magnetischen Partikeln oder alternativen Bindungsverfahren, der Inkubation bei einer bestimmten Temperatur, der Entfernung nicht absorbierter Probenbestandteile, den Waschschritten, der Elution gebundener Nukleinsäuren von den Partikeln bei einer bestimmten Temperatur und dem Abtrennen des Eluats von der Partikelsuspension Anwendung findet.

Der Begriff "Abtrennen" umfasst die soweit wie mögliche Entfernung von allen biologischen oder chemischen Substanzen oder Komponenten, die nicht das eigentliche Ziel der Isolation - also im Wesentlichen keine Nukleinsäuren - sind. Insbesondere dient die Abtrennung dieser Substanzen der Vermeidung von Interferenzen oder Störeinflüssen während der eigentlichen Bindung, Anreicherung, Aufreinigung und anschließenden Detektion von den Zielmolekülen.

Der Begriff "zelluläres Debris" umfasst sämtliche biologischen Komponenten, die nicht primäres Target einer Nukleinsäureisolation sind und durch einen Reinigungs- oder Negativselektionsschritt von den eigentlichen Zielmolekülen getrennt werden sollen. Nach erfolgter Lyse einer zellulären Probe gehören dazu insbesondere in wässriger Lösung unlösliche und schwer lysierbare Zellbestandteile wie z.B. nekrotisierende Gewebebestandteile, Knochen- oder Kalkstrukturen, insbesondere Mikrocalzifizierungen, des weiteren aber auch geplatzte oder morphologische veränderte Erythrocyten, warzen- und papillomartige Gewebestrukturen, sowie auch spezielle Bakterien, welche eine komplexe, schwer lysierbare Zuckerhülle aufweisen (z.B. Mycobakterien). Des Weiteren gehören Proteine, Membranbestandteile, insbesondere durch Fixierung vernetzte Strukturen etc dazu. In Einzelfällen kann es sich aber auch um in Wasser lösliche Komponenten handeln, welche nach den oben beschriebenen Lyseprozessen freigesetzt werden und abgetrennt werden sollen. Ein Beispiel ist das in großen Mengen und molarem Überschuss gegenüber Nukleinsäuren freigesetzte Hämoglobin nach der Lyse (z.B. durch hypotonische Pufferbedingungen) von Erythrozyten, welches vor der weiteren Verarbeitung der Körperprobe abgetrennt werden soll.

Darüber hinaus sind mit "zelluläres Debris" insbesondere alle Komponenten im Faeces/Stuhl gemeint, die keine Nukleinsäuren sind. Stuhl besteht zum einen aus den unverdaulichen Anteilen der Nahrung (Ballaststoffe) sowie unverdauten Resten wie Fett, Stärke, Bindegewebs- und Muskelfasern und Wasser, die nicht in den oberen Dickdarmabschnitten resorbiert wurden. An körpereigenen Substanzen sind enthalten: abgestoßene Darmzellen mit Nukleinsäuren, die isoliert werden sollen, Rückstände von Verdauungsenzymen und Schleim. Des Weiteren werden geringe Mengen der Gallensäuren selbst, sowie des zum Schutz der Darmschleimhaut ebenfalls von der Galle ausgeschiedenen Lecithins und anderer Phospholipide zusammen mit dem Kot ausgeschieden.

Der Begriff "magnetische Partikel" umfasst sowohl organische als auch anorganische magnetische Partikel.

Der Begriff "Silika" umfasst Siliziumdioxid und Siliziumdioxidderivate, insbesondere SiO₂ Kristalle und andere Formen von SiO₂, z.B. mit SiO₂ aufgebaute Diatomeen, Zeolithe, amorphes Siliziumdioxid, Glaspulver, Kieselsäure, Wasserglas sowie Aluminiumsilikate und aktivierte Silikate.

Der Begriff "hydrophobe Matrix" bezeichnet eine Festphase, deren Oberfläche mit einem hydrophoben Material aufgebaut ist, insbesondere ein hydrophobes Kunststoffmaterial, z.B. Polyolefinen, wie PP (Polypropylen), PE (Polyethylen), halogenierte Polyolefine, wie z.B. PTFE (polytetrafluorethylen), und andere. Die Matrix kann in jeder geeigneten Form vorliegen, z.B. in Form von Partikeln, Fasern, flächige Oberflächen usw. Insbesondere kann die Matrix in Form einer Gefäßinnenwand ausgebildet sein.

Der Begriff "Lysepuffersystem" beinhaltet ein Puffersystem, welches mindestens eine Substanz enthält, die den Aufschluss einer Zelle, eines Zellsystems, Zellbestandteilen oder anderen biologischen Komplexen bzw. Strukturen bewirken oder begünstigen kann. Besonders häufig sind die Substanzen ausgewählt aus der Gruppe von Detergenzien (Triton X-100, SDS, oder ähnliches) sowie enzymatische Reagenzien wie insbesondere Proteinase K. Mit umfasst ist die Verwendung von Reagenzien aus der Gruppe von wässrigen, gepufferten oder ungepufferten Lösungen (im einfachsten Fall Wasser). In einem Lysepuffersytem können ein oder mehrere Komponenten aus einer oder beiden Gruppen oder miteinander kombiniert werden. Im Rahmen dieser Erfindung sind Reagenzien, die chaotrope Substanzen enthalten ausdrücklich nicht als Bestandteil des Lysepuffersystems in den ersten Schritten einer Aufreinigung gemeint.

Die weiteren in der vorliegenden Anmeldung verwendeten Begriffe haben die gewöhnliche, dem Fachmann bekannte Bedeutung.

### Zusammenfassung der Erfindung

Die Erfindung umfasst Verfahren zum Aufreinigen von Nukleinsäuren aus einer biologischen Probe, die dadurch gekennzeichnet sind, dass nach Aufnehmen der Probe in wässriger Lösung und nach Lysieren der Probe Debris aus der Probe unter nicht-chaotropen Bedingungen abgetrennt wird, bevor die Nukleinsäuren dann aus dem von Debris gereinigten Lysat isoliert werden.

Die Erfindung betrifft ein Verfahren zum Aufreinigen von Nukleinsäuren aus einer biologischen Probe, aufweisend die folgenden Schritte:
a) Aufnehmen der Probe in einer wässrigen Lösung;
b) Lysieren der Probe;
c) Abtrennen von zellulärem Debris durch Suspendieren von magnetischen Partikeln in der Lösung und Anwenden eines magnetischen Feldes zum Zurückhalten der magnetischen Partikel und Abtrennen der magnetischen Partikel von der Lösung; und
d) Isolieren der Nukleinsäuren aus der Lösung;
wobei Schritte (a) bis (c) unter nicht-chaotropen Bedingungen stattfinden.

Die Erfinder haben gefunden, dass das Abtrennen von zellulärem Debris unter nicht-chaotropen Bedingungen die weitere Isolierung der Nukleinsäuren vorteilhaft vereinfacht und insbesondere auch zu einer verbesserten Automatisierbarkeit der Aufreinigungsverfahren führt. Allgemeiner ausgedrückt betrifft die Erfindung ein Verfahren zum Aufreinigen von Nukleinsäuren aus einer biologischen Probe, aufweisend die folgenden Schritte:
a) Aufnehmen der Probe in einer wässrigen Lösung;
b) Lysieren der Probe;
c) Abtrennen von zellulärem Debris; und
d) Isolieren der Nukleinsäuren aus der Lösung;
wobei Schritte (a) bis (c) unter nicht-chaotropen Bedingungen stattfinden.

Gemäß einem Aspekt der vorliegenden Erfindung umfasst der Schritt (c) insbesondere das Suspendieren von magnetischen Partikeln in der Lösung und Anwenden eines magnetischen Feldes zum Zurückhalten der magnetischen Partikel und Abtrennen der magnetischen Partikel von der Lösung. Generell könnte auch die Zugabe von Partikeln generell, also auch nichtmagnetische Partikel anstatt der magnetischen Partikel (z.B. Zellulosepartikel, Keramikpartikel, Kunststoffpartikel) vorteilhaft sein, da sie eine bessere Aggregierung des Debris fördern.

Die magnetischen Partikel haben gemäß einem Aspekt der Erfindung eine mittlere Größe von <50 µm, bevorzugt <10 µm, ganz bevorzugt <0,5 µm, nicht ausschließend < 0.1 µm, wobei die Größe durch Transmissionselektronmikroskpieverfahren bestimmt wird.

Gemäß einem Aspekt der Erfindung weisen diese Partikel eine siliziumhaltige Beschichtung, insbesondere eine Siliziumdioxid aufweisende Beschichtung auf. Derartige magnetische Partikel sind beispielsweise bekannt aus EP 1468430.

Die Magnetpartikel weisen bevorzugt eine Silikabeschichtung auf, sind also SiO₂ umhüllte magnetische Partikel. Der Ausdruck "SiO₂-umhüllte magnetische Partikel" umfasst Magnetit-Kerne, die aus mindestens 90 Gewichtprozent Fe₃O₄ bestehen und deren Oberfläche mit Silikat beschichtet ist.

Die magnetischen Partikel sind suspendierbare Partikel, welche durch Anwenden eines externen Magnetfeldes in dem Magnetfeld immobilisiert werden können.

Das Abtrennen der magnetischen Partikel von der Lösung erfolgt nach Immobilisierung der Partikel durch das Magnetfeld, die Lösung kann dann auf jede geeignete Weise von den Partikeln getrennt werden, z.B. durch Abkippen, Absaugen, etc..

Das Isolieren der Nukleinsäuren aus der Probe gemäß Schritt (d) kann mit jedem geeigneten Verfahren erfolgen, z.B. Extraktionsverfahren, säulenbasierte Verfahren, Ausfällen etc.. Der Schritt (d) des Isolierens der Nukleinsäuren ist auf keinen bestimmten Reinheitsgrad der isolierten Nukleinsäuren beschränkt.

In Schritt (a) wird die Probe in einer wässrigen Lösung aufgenommen. Dies kann durch Mischen, Suspendieren, Emulgieren oder Auflösen geschehen. Die Probe kann vor oder nach dem Aufnehmen in wässriger Lösung mechanisch verkleinert werden, beispielsweise durch mechanische Einwirkung (z.B. zerschneiden, rühren), durch Hitzeeinwirkung, durch Ultraschallbehandlung und ähnliche Verfahren. Es ist aber auch möglich, die intakte Gewebeprobe, z.B. einen Gewebeschnitt, direkt in wässriger Lösung zu suspendieren.

Gemäß einem Aspekt der Erfindung ist die Probe eine Blutprobe. Bei der Komplettlyse von Blut wird in großen Mengen und molarem Überschuss gegenüber den Nukleinsäuren Hämoglobin und auch Erythrozyten- sowie Leukozytenmembranbestandteile freigesetzt, welche unter Schritt c) abgetrennt werden können. Der Schritt d) dient dann zur gezielten Aufreinigung der Nukleinsäuren aus der wässrigen Restphase.

Alternativ kann man wie bereits technisch beschrieben selektive Lyse von Erythrozyten durchführen unter z.B. hypotonischen Pufferbedingungen. Dabei werden Hämoglobin und Erythrozytenmembranen in großen Mengen freigesetzt, welche unter Schritt c) abgetrennt werden können. In diesem Falle würde in Schritt d) bei Zugabe eines chaotropen Puffers und evtl. Proteinase K die noch notwendige Leukozytenlyse mit Freisetzung der Nukleinsäuren erfolgen. Diese Verfahrensweisen ermöglichen eine einfache und vollständige Automatisierung der Extraktion von Nukleinsäuren aus Blut, insbesondere Leukozyten, wobei aufwendige Verfahrensschritte wie Zentrifugation und Pelletierung der Leukozyten und Verwerfung des Überstandes vermieden werden können.

Gemäß einem weiteren Aspekt der Erfindung ist die Probe eine Stuhl-/Faecesprobe. Bei der Lyse von Stuhl werden Nukleinsäuren aus gesunden oder krankhaft veränderten, ausgeschiedenen Darmepithelzellen freigesetzt in eine komplexe Matrix aus Zelltrümmern, Ballaststoffen, unverdauten Nahrungsresten wie Fett, Stärke, Bindegewebs- und Muskelfasern, Rückstände von Verdauungsenzymen und Schleim, Gallensäuren, Lecithine und andereren Phospholipiden. All diese Komponenten, die keine Nukleinsäuren sind, können unter Schritt ganz oder teilweise c) abgetrennt werden. Der Schritt d) dient dann zur gezielten Aufreinigung der Nukleinsäuren aus der wässrigen Restphase.

Gemäß einem Aspekt der Erfindung ist die biologische Probe eine in Paraffin eingebettete Probe, insbesondere ein Paraffinschnitt, und/oder eine fixierte Probe, insbesondere ein formalinfixierter Paraffinschnitt.

Das erfindungsgemäße Verfahren eignet sich besonders für die Aufarbeitung fixierter Proben, da fixierte Proben z.B. aufgrund von Protein- und Nukleinsäurevernetzung besonders viel Debris aufweisen.

Gemäß einem bevorzugten Aspekt der Erfindung wird die Lösung vor dem Schritt (d) auf mindestens 50°C, bevorzugt 50 - 95 °C, bevorzugt mindestens 60 °C, stärker bevorzugt 60 - 80°C, erhitzt. Dieses Erhitzen ermöglicht vorteilhaft eine bessere Suspension und eine verbesserte Lyse der biologischen Probe in der wässrigen Lösung. Zur effektiveren Lyse ist es bevorzugt, eine Proteinase zuzugeben, insbesondere Proteinase K.

Gemäß einem Aspekt der Erfindung wird vor dem Schritt (c) die Probe wiederum auf unterhalb 50°C abgekühlt. Bei Vorhandensein von Paraffin in der Probe hat das Abkühlen auf unterhalb 50°C den zusätzlichen Vorteil, dass sich das Paraffin wieder verfestigt, z.B. in Form eines Paraffinringes an der Gefäßwand. Die Probe bzw. das Lysat kann dann beispielsweise mit einer Pipettenspitze sehr einfach und akkurat ohne Verstopfungsprobleme abgesaugt werden, wobei das Paraffin in Form des beschriebenen Paraffinringes im Reaktionsgefäß zurückbleibt.

Gemäß einem weiteren Aspekt der Erfindung wird die Probe bzw. das Lysat mit einer hydrophoben Matrix in Kontakt gebracht, in dem sie z.B. in einem Gefäß aus hydrophobem Kunststoffmaterial aufgenommen wird. Dies ist besonders bei der Aufarbeitung von paraffinhaltigen Proben bevorzugt. Als hydrophobe Matrix geeignet sind hierzu beispielsweise die bekannten Reaktionsgefäße von Eppendorf oder Sarstedt, welche aus Polyolefinen (z.B. Polypropylen und Polyethylen) bestehen. Es ist besonders bevorzugt, Paraffin enthaltende Proben in Kontakt mit einer hydrophoben Matrix vor Schritt (d) auf über 50°C zu erwärmen, weil dadurch das Paraffin schmilzt und sich bei Abkühlung vorteilhaft als Ring an der Flüssigkeitsoberfläche an der Matrix, z.B. bei einem Kunststoffreaktionsgefäß am Gefäßrand absetzt. Dies geschieht durch Absorptionsvorgänge des verflüssigten Paraffins an der hydrophoben Matrix. In Folgeschritten kann deswegen vorteilhaft anschließend die flüssige Probe akkurat ohne Verstopfungen von Pipettenspitzen abgesaugt werden, während der Paraffinring im Reaktionsgefäß zurückbleibt.

Gemäß eines weiteren Aspekts der Erfindung ist das beschriebene Verfahren bzgl. der Aufreinigungseffizienz so gut, dass für die meisten Anwendungen ein einzelner 3 - 20 µm, ganz besonders bevorzugt ein einzelner 10 µm Paraffinschnitt ausreicht, um sehr hohe Ausbeuten von Nukleinsäuren zu erzielen. Dadurch ist die eingesetzte Paraffinmenge unterhalb von kritischen Mengen, welche eine Ausbildung des Ringes verhindern oder stören.

Gemäß einem Aspekt der Erfindung wird die Lösung in Schritt (c) durch Absaugen von den Magnetpartikeln abgetrennt.

Gemäß einem Aspekt der Erfindung umfasst Schritt (d) ferner das Hinzufügen einer chaotropen Verbindung zu der Lösung. Dabei ist die erstmalige oder wiederholte Hinzugabe von Proteinase K (falls in Schritt a) schon Proteinase K verwendet wurde) in Schritt d), d.h. bevor oder nach der Zugabe der chaotropen Lösung nicht ausgeschlossen.

Gemäß einem Aspekt der vorliegenden Erfindung umfasst Schritt (d) ferner das Hinzufügen von unverbrauchten (frischen) magnetischen Partikeln mit siliziumhaltiger Beschichtung zu der Lösung.

Zur Isolation von RNA ist es bevorzugt, eine DNase in biologisch wirksamer Menge zu der Probe hinzuzufügen. Dadurch wird DNA "verdaut" und geht in Lösung, während die unverdaute RNA aus der Lösung isoliert werden kann. Der DNase Verdau kann zu unterschiedliche Zeitpunkten der Extraktion durchgeführt werden, frühestens nach der Lyse und spätestens nach der Elution am Ende der Aufreinigung.

Zur Aufreinigung von DNA ist es bevorzugt, zu der Probe RNase in biologisch wirksamer Menge hinzuzufügen, wodurch RNA verdaut und die intakte DNA aus der Probe isoliert werden kann. Der RNase Verdau kann zu unterschiedliche Zeitpunkten der Extraktion durchgeführt werden, frühestens nach der Lyse und spätestens nach der Elution am Ende der Aufreinigung. Bevorzugt ist allerdings den DNA Nachweis in Gegenwart der mitaufgereinigten RNA zu führen, also unter Weglassen des RNase Schrittes oder unter Verwendung von Pufferbedingungen, welche eine selektive Isolierung von DNA unter Ausschluss der RNA ermöglichen.

Die Erfindung umfasst ferner die Verwendung von Magnetpartikeln mit siliziumhaltiger Beschichtung zur Entfernung von zellulärem Debris aus einer biologischen Probe unter nicht-chaotropen Bedingungen. Insbesondere können die Magnetpartikeln mit siliziumhaltiger Beschichtung Magnetpartikel mit Silika enthaltender Beschichtung sein.

Im Folgenden wird die Erfindung beschrieben anhand von detaillierten Beispielen im Zusammenhang mit den Figuren, in welchen gezeigt ist:
- FIG 1:: eine schematische Darstellung einer Ausführungsform des erfindungsgemäßen Verfahrens (entweder manuell oder automatisiert)
- FIG 2:: einen Vergleich der Ausbeute zwischen einem ersten manuell durchgeführten erfindungsgemäßen Verfahren mit Entfernung von Debris durch Zentrifugation (a), einem zweiten automatisiert durchgeführten erfindungsgemäßen Verfahren mit Entfernung von Debris durch magnetische Partikel (b), und einem Verfahren gemäß dem Stand der Technik (Qiagen RNeasy FFPE Kit) ohne vorherige Entfernung von Debris (c).
- FIG 3:: einen Vergleich(Korrelation) der Ausbeute zwischen einem ersten manuell durchgeführten erfindungsgemäßen Verfahren mit Entfernung von Debris durch Zentrifugation (a) und einem Verfahren gemäß dem Stand der Technik (Qiagen RNeasy FFPE Kit) ohne vorherige Entfernung von Debris (c).
- FIG 4:: einen Vergleich der Ausbeute von 2 manuellen Aufreinigungen nach dem erfindungsgemässen Verfahren mit Zentrifugation bzw. mit zusätzlichem Beadbindungsschritt für Debris sowie nach einem manuellen Verfahren gemäß Stand der Technik
- FIG 5:: Vergleich des automatisierten erfindungsge mässen Verfahrens mit einem automatisierten Verfahren aus dem Stand der Technik
- Fig. 6: Total Aspiration and Dispense Monitoring (TADM) Kurven nach dem automatisierten erfindungsgemässen Verfahrens
- Fig. 7: Total Aspiration and Dispense Monitoring (TADM) Kurven nach einem automatisierten Ver fahren gemäss Stand der Technik

Bei üblichen Verfahren der Nukleinsäureaufreinigung wird das Probenmaterial in einem chaotropen Puffer aufgenommen bzw. lysiert. Die vorliegende Erfindung basiert auf der überraschenden Erkenntnis, dass eine Nukleinsäureaufreinigung zu verbesserten Ergebnissen führt, wenn vor dem Isolieren der Nukleinsäuren aus der Probe zelluläres Debris unter nicht-chaotropen Bedingungen entfernt wird. Dies kann beispielsweise durch Zentrifugation oder Filtrieren geschehen. Gemäß einer Ausführungsform der Erfindung wird das Debris mit Hilfe von magnetischen Partikeln unter nicht-chaotropen Bedingungen entfernt. Bevorzugt weisen diese Partikel eine siliziumhaltige Beschichtung, insbesondere eine Siliziumdioxid aufweisende Beschichtung auf. Solche Partikel sind bekannt aus EP 1468430, welche durch Bezugnahme hierin inkorporiert wird. Die Herstellung derartiger Partikel wird weiter unten ausführlich beschrieben.

Die Isolierung von Nukleinsäuren aus dem von Debris gereinigten Lysat kann durch bekannte Verfahren erfolgen. Geeignet sind beispielsweise Extraktionsprotokolle, die auf einer Aufreinigung aus chaotropen Lösungen basieren, z.B. durch Fällung der Nukleinsäuren und/oder Absorption an silikahaltige Matrices unter chaotropen Bedingungen. Bei bekannten säulenbasierten Verfahren werden aus dem Lysat die Nukleinsäuren in Gegenwart chaotroper Salze, die in hoher Konzentration zugegen sind, an eine Silika-Membran gebunden und nach einem Reinigungsschritt von der Membran eluiert. Entsprechende Kits sind von der Fa. QIAGEN GmbH, Hilden, Bundesrepublik Deutschland, kommerziell erhältlich.

Gemäß einem bevorzugten Aspekt der Erfindung erfolgt die Isolierung der Nukleinsäuren durch eine erneute Anwendung von (frischen) silikabeschichteten Magnetpartikeln unter chaotropen Bedingungen.

Gemäß einem bevorzugten Aspekt der Erfindung ermöglicht die Abtrennung von Debris bzw. störenden Substanzen auch die Erhöhung der Aufreinigungseffizienz, Reproduzierbarkeit und Robustheit, sowie die Reduzierung von Ausreißern und nicht eindeutigen bzw. unklaren Ergebnissen in der Analyse der Nukleinsäuren ("Flagging" von Ergebnissen, "geflagte" Resultate). Dies geht in der klinischen Diagnostik, wo solche Verfahren neben der Forschung auch eingesetzt werden sollen, einher mit sogenannten Wiederholungs- bzw. Reflextests, die zusätzliche und vermeidbare Kosten bedeuten.

### BEISPIELE:

### Material und Methoden:

### Folgende Materialien und Methoden wurden in allen folgenden Beispielen verwendet

Als Ausgangsmaterial dienen Tumorproben aus einem klinischpathologischen Labor, welche zum Zeitpunkt der Entnahme in Formalin fixiert und anschließend in Paraffin eingebettet wurden. Diese Verfahren zur Fixierung und Einbettung sind dem Fachmann allgemein bekannt und werden hier nicht näher beschrieben. Mit einem Mikrotom werden von der Probe Gewebeschnitte, z.B. mit einer Dicke von 5 bis 10 µm, erhalten und in ein 1,5 ml Probengefäß überführt, z.B. ein 1,5 ml Polypropylen-Probengefäß. Alternativ können auch Proben, welche bereits auf einen Objektträger aufgebracht wurden, von diesem mit einer Rasierklinge oder auf andere geeignete Weise (z.B. Entparaffinisierung mit Ethanol/Xylol)abgelöst oder heruntergekratzt und in das Probengefäß überführt werden.

Neben den kommerziell erhältlichen "Versant kPCR Sample Preparation Reagents der Firma Siemens Healthcare Diagnostics GmbH (Erlangen, Deutschland) (bestehend aus einer Proteinase K Lösung, Bindungspuffer (enthält Chaotrop z.B. 59% Guanidinthiocyanat und 10% Octylphenoxypolyethoxyethanol), silicabeschichtete magnetische Partikel, wie z.B. aus EP 1468430 bekannt, Waschpuffer 1 (enthält Chaotrop bzw. 36% Guanidinthiocyanat und 30% Ethanol, Waschpuffer 2 (enthält 80% Ethanol), Waschpuffer 3 (enthält 5-Chlor-2-methyl-4-isothiazolin-3-on und 2-Methly-4-isothiazolin-3-on (3:1)) und Elutionspuffer (enthält Natriumazid) wurden folgende Puffer verwendet:
1. FFPE Lysepuffer
   10 mM Tris-HCl
   0.1 mM EDTA
   2% SDS
   pH 8.0
2. DNA-free DNAse Lösung(Ambion, Cat# A 1906, Ambion, Foster City, CA 94404, USA)

Anstatt der Puffer der kommerziell erhältlichen "Versant kPCR Sample Preparation Reagents" der Firma Siemens Healthcare Diagnostics GmbH können auch andere übliche, dem Fachmann bekannte Pufferzusammensetzungen gewählt werden. Als FFPE Lysepuffer kommen insbesondere detergenzhaltige und/ oder hypotone Puffer in Betracht. Geeignete Waschpuffer sind ebenfalls aus dem Stand der Technik bekannt und kommerziell erhältlich. Als Bindungspuffer für die nachfolgende Isolierung von Nukleinsäuren mit silica-beschichteten Magnetbeads aus dem Lysat kommen chaotrope Pufferzusammensetzungen in Frage, z.B. 4.5 M Guanidinium HCl, 6M Guanidiniumisothiocyanat u. Ä.. Die Anforderungen, die an geeignete Waschpuffer zu stellen sind, bestehen lediglich darin, dass der Puffer gewährleisten muss, dass die Nukleinsäure nicht von der Silica-Matrix abgelöst wird. Allgemein genügt ein hoher Alkoholgehalt und optional leicht alkalischer pH, um die Autoproteolyse der DNA zu verhindern. Auch Waschpuffer, die chaotrope Verbindungen enthalten sind geeignet, solange sie die oben erwähnten Bedingungen erfüllen. Als Elutionspuffer kommen auch dem Fachmann bekannte Pufferzusammensetzungen in Frage, z.B. TE Puffer (10 mM Tris-HCl, 0.1 mM EDTA, pH 8.0).

Es wird darauf hingewiesen, dass in diesem und ähnlichen Aufreinigungsprotokollen die RNA zu 100 bis 500 Basenpaaren langen Fragmenten fragmentieren kann, für Expressionsanalyse mit gängigen Verfahren (RT-PCR, Micro-Array u.ä.) ist jedoch auch fragmentierte RNA gut geeignet.

Die (relative) Quantifizierung RNA-Ausbeute fand mittels der einstufigen kinetischen Real-Time Reverse Transkriptase Polymerase Kettenreaktion (One-Step kRT-PCR) mit Hilfe einer TaqMan Sonde statt. Zur Analyse der RNA Ausbeute wurde der CT-Wert (cycle treshold, d.h. der Wert des Amplifikationszyklusses, welcher als erster einen definierten Schwellwert übersteigt) für die RNA des Referenz- bzw. Housekeeping-Gens RPL37A bestimmt, d.h. die mRNA des humanen Gens für ribosomales Protein L37a, Genbank-Accesion Number NM_000998. Die q RT-PCR wurde unter Verwendung des "SuperScript™ one-step with a Platinum® Taq kits" der Firma Invitrogen, Karlsruhe, Deutschland, und unter Verwendung von Primern und einer Sonde der Firma Eurogentec, Köln, Deutschland durchgeführt.

Zur Durchführung einer kRT-PCR Expressionsanalyse von RPL37A wurde 1 µl aufgereinigte RNA zu 9 µl Mastermix hinzugegeben, bestehend aus 400 nM forward primer, 400 nM reverse primer, 200 nM Taqman-Sonde (FAM/ TAMRA markiert), Reaktionsmix mit jeweils 0,2 mM dNTP und 1,2 mM Magnesiumsulfat sowie 1 µl Platinum® Taq Mix. Die Reaktion wurde auf einem ABI7900 Gerät der Firma Applied Biosystems, Applera Deutschland GmbH, Darmstadt, Deutschland, mit folgendem Temperatur-Profil durchgeführt:
- 30 min. bei 50°C
- 2 min. bei 95°C
- 15 sek. bei 95°C
- 30 sek. bei 60°C, 40 Zyklen

Zur Ermittlung der CT-Werte wurde die Software SDS 2.0 von Applied Biosystems entsprechend der Betriebsanweisung verwendet. Der CT-Wert entspricht der Anzahl von Amplifikationszyklen, ab welcher das Amplifikationssignal eine definierte Schwelle, z.B. die messschwelle, überschritten hat. Je mehr Transkript in der Probe vorliegt, um so geringer ist dementsprechend der CT Wert. Zum Teil wurden die CT-Werte für RPL37A in den Abbildungen zwecks besserer Darstellung als 40-CT angegeben. Auf diese Weise werden die Werte invertiert und höhere 40-CT Werte entsprechen höheren Expressionswerten von RPL37A. Wenn nicht näher spezifiziert ist der direkt gemessene CT-Wert gemeint.

### Beispiel 1: Aufreinigung von Nukleinsäuren aus Paraffinschnittproben mit Entfernung von Debris durch Zentrifugation unter nicht-chaotropen Bedingungen.

RNA aus FFPE-Gewebe Schnitten wurden folgendermaßen manuell aufgereinigt:
- FFPE-Gewebe Schnitt in einem Eppendorf-Probegefäß 1 min bei maximaler Geschwindigkeit zentrifugieren
- Aufnehmen des Paraffinschnittes in 150 µl FFPE Lysepuffer und 50 µl Proteinase K-Lösung;
- Inkubation für 2 Stunden bei 65°C unter Schütteln;
- Zentrifugation (z.B. 5 min. bei 10.000 g) bei Raumtemperatur, vorsichtiges Übertragen des flüssigen Überstands in ein frisches Probengefäß (dadurch Abtrennen von Debris und Paraffinresten);
- Hinzufügen von 800 µl Bindungspuffer und Durchmischen der Lösung
- Hinzufügen von 50 µl einer Suspension von magnetischen Partikeln zu der Probe und Durchmischen der Lösung
- Inkubation für 15 min. bei Raumtemperatur unter Schütteln;
- Anlegen eines Magnetfeldes, Absaugen und Verwerfen des Überstands;
- Entfernen des Magnetfeldes. Aufnehmen und Suspendieren der magnetischen Partikel (mit den daran gebundenen Nukleinsäuren) in 850 µl Waschpuffer 1;
- Anlegen des Magnetfeldes, Absaugen und Verwerfen des Überstands;
- Entfernen des Magnetfeldes. Aufnehmen und Suspendieren der magnetischen Partikel (mit den daran gebundenen Nukleinsäuren) in 450 µl Waschpuffer 2;
- Anlegen des Magnetfeldes, Absaugen und Verwerfen des Überstands;
- Entfernen des Magnetfeldes. Aufnehmen und Suspendieren der magnetischen Partikel (mit den daran gebundenen Nukleinsäuren) in 450 µl Waschpuffer 3;
- Wiederholtes Waschen mit Waschpuffer 3;
- Nach Anlegen des Magnetfeldes und Entfernen des Überstandes aufnehmen der Probe in 100 µl Elutionspuffer, Inkubation 10 min. bei 70°C unter Schütteln in einem Thermomixer;
- Anlegen eines Magnetfeldes, Überführen des Eluats in ein frisches Probengefäß.
- Zugabe von 10 µl 10x DNAse Puffer und 1 µl DNAseI
- 30 min bei 37°C inkubieren

Einfrieren der Proben und/ oder weitere Analyse der Eluate.

### Beispiel 2: Isolierung von RNA aus einem formalinfixierten Paraffinschnitt mit Entfernung von Debris durch Zugabe von magnetischen Partikeln unter nicht-chaotropen Bedingungen.

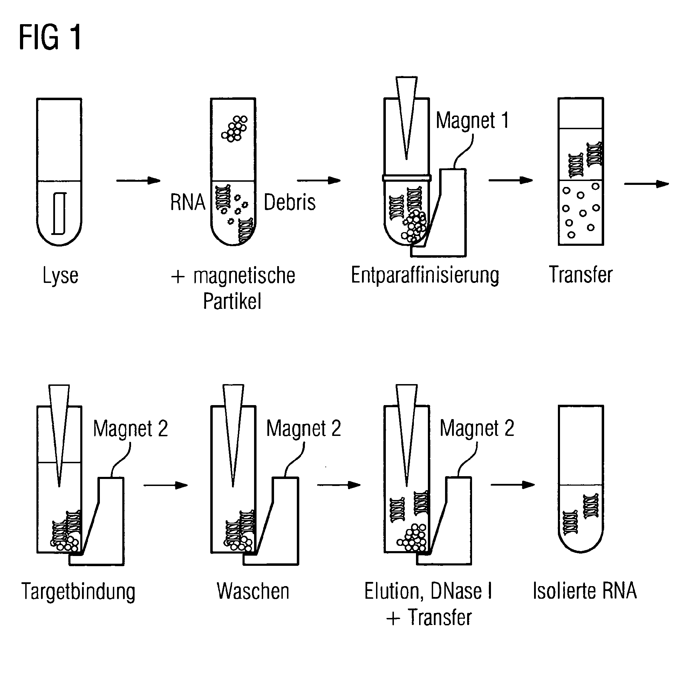

Dieses Verfahren entspricht dem in Fig. 1 schematisch dargestellten Verfahren.

RNA aus FFPE-Gewebe Schnitten wurden folgendermaßen manuell aufgereinigt:
- FFPE-Gewebe Schnitt in einem Eppendorf-Probegefäß 1 min bei maximaler Geschwindigkeit zentrifugieren
- Zugabe von 150 µl FFPE Lysepuffer und 50 µl Proteinase K
- 2 h bei 65°C unter Schütteln inkubieren
- Zugabe von 50 µl magnetischer Partikel
- Mischen durch 2 min Schütteln
- Probe magnetisieren
- Überstand vorsichtig in ein neues Gefäß überführen (Zell Debris und Paraffin-Reste verbleiben im alten Gefäß)
- Zugabe von 800 µl Bindungspuffer (Chaotrop)
- Zugabe von 50 µl magnetischer Partikel
- Inkubation für 15 min. bei Raumtemperatur unter Schütteln;
- Anlegen eines Magnetfeldes, Absaugen und Verwerfen des Überstands;
- Entfernen des Magnetfeldes. Aufnehmen und Suspendieren der magnetischen Partikel (mit den daran gebundenen Nukleinsäuren) in 850 µl Waschpuffer 1;
- Anlegen des Magnetfeldes, Absaugen und Verwerfen des Überstands;
- Entfernen des Magnetfeldes. Aufnehmen und Suspendieren der magnetischen Partikel (mit den daran gebundenen Nukleinsäuren) in 450 µl Waschpuffer 2;
- Anlegen des Magnetfeldes, Absaugen und Verwerfen des Überstands;
- Entfernen des Magnetfeldes. Aufnehmen und Suspendieren der magnetischen Partikel (mit den daran gebundenen Nukleinsäuren) in 450 µl Waschpuffer 3;
- Wiederholtes Waschen mit Waschpuffer 3;
- Nach Anlegen des Magnetfeldes und Entfernen des Überstandes aufnehmen der Probe in 100 µl Elutionspuffer, Inkubation 10 min. bei 70°C unter Schütteln in einem Thermomixer;
- Anlegen eines Magnetfeldes, Überführen des Eluats in ein frisches Probengefäß.
- Zugabe von 10 µl 10x DNAse Puffer und 1 µl DNAseI
- 30 min bei 37°C inkubieren

Einfrieren der Proben und/ oder weitere Analyse der Eluate.

In Abbildung 1 ist das erfindungsgemäße Verfahren schematisch dargestellt. Es kann manuell oder automatisiert ablaufen.

Beispiel 3: Automatisierte Aufreinigung von RNA aus formalinfixierten Paraffinschnitten.

RNA aus formalinfixierten Paraffinschnitten wurde aufgereinigt unter Verwendung des folgenden automatisierten Protokolls auf einer Siemens Plattform VERSANT kPCR (Extraktionseinheit). Bis zu 48 Gewebeschnitte können in einem Durchlauf aufgereinigt werden.

### Probenvorbereitung

Gewebeschnitte (5 - 10 µl) wurden durch Zentrifugation bei Raumtemperatur pelletiert und auf Probenträger der Siemens Molekularen Plattform VERSANT kPCR platziert, wo sämtliche Hardwaremodule (Heater/Shaker, Magneten etc), Probengefäße, Puffer und Pipettenspitzen an ihren designierten Positionen platziert sind.

Start Aufreinigungsprogramm:
- Hinzufügen von 150 µl Lysepuffer zu Proben;
- Hinzufügen von 50 µl Proteinase K-Lösung;
- Übertragen der Probengefäße auf einen Heizschüttler und Inkubation für 2 Stunden bei 65°C unter Schütteln
- Transfer der Lysatflüssigkeit zu einer Deep-well Probenplatte (DWP)
- Hinzufügen von 600 µl Bindungspuffer (Chaotrop);
- Hinzufügen von 50 µl Magnetpartikelsuspension in die DWP;
- Inkubation für 10 min. bei Raumtemperatur unter Schütteln;
- Transfer der DWP zu Magneten;
- Inkubation 5 min. bei Raumtemperatur im Magnetfeld;
- Absaugen und Verwerfen des Überstands;
- Transfer der DWP von Magnet zum Heizschüttler;
- Hinzufügen von 850 µl Waschpuffer 1;
- 10 sek. Schütteln bei Raumtemperatur;
- Transfer der DWP zum Magneten;
- Magnetisierung 2 min. bei Raumtemperatur;
- Absaugen und Verwerfen des Überstands;
- Transfer der DWP vom Magneten zum Heizschüttler;
- Hinzufügen von 450 µl Waschpuffer 2;
- 10 sek. Schütteln bei Raumtemperatur;
- Transfer der DWP zum Magneten;
- Magnetisierung 2 min. bei Raumtemperatur;
- Absaugen und Verwerfen des Überstands;
- Transfer der DWP von Magnet zum Heizschüttler;
- Hinzufügen von 850 µl Waschpuffer 3;
- 10 sek. Schütteln bei Raumtemperatur;
- Transfer der DWP zum Magneten;
- Magnetisierung 2 min. bei Raumtemperatur;
- Absaugen und Verwerfen des Überstands;
- Hinzufügen von 100 µl Elutionspuffer;
- Transer der DWP von Magnet zum Heizschüttler;
- Inkubation 10 min. bei 70°C unter Schütteln;
- Tranfer der DWP zum Magneten;
- Hinzufügen von 12 µl DNase-Mix (10 µl 10x DNase-Puffer; 2 µl DNase 1);
- Transfer der DWP von Magnet zum Heizschüttler (heruntergekühlt auf 37°C);
- Inkubation 30 min. bei 37°C ohne Schütteln;
- Transfer der DWP zu Magnet;
- Übertragen der DNase-verdauten Proben auf 1,5 ml Probengefäße; Ende Aufreinigungsprogramm
- Einfrieren der Proben und/ oder weitere Analyse der Eluate

### Beispiel 4: Automatisierte Aufreinigung von RNA aus formalinfixierten Gewebeschnitten unter Verwendung eines zusätzlichen Bindungsschritts mit magnetischen Partikeln zum Abtrennen von zellulärem Debris unter nicht-chaotropen Bedingungen

Fig. 1 zeigt die schematische Darstellung des erfindungsgemäßen Verfahrens.

RNA aus formalinfixierten Paraffinschnitten wurde aufgereinigt unter Verwendung des folgenden automatisierten Protokolls auf einer Siemens Plattform VERSANT kPCR (Extraktionseinheit). Bis zu 48 Gewebeschnitte können in einem Durchlauf aufgereinigt werden.

### Probenvorbereitung

Gewebeschnitte (5 - 10 µm) wurden durch Zentrifugation bei Raumtemperatur pelletiert und auf Probenträger der Siemens Molekularen Plattform VERSANT kPCR platziert, wo sämtliche Hardwaremodule (Heater/Shaker, Magneten etc), Probengefäße, Puffer und Pipettenspitzen an ihren designierten Positionen platziert sind.

### Start Aufreinigungsprogramm:

- Hinzufügen von 150 µl Lysepuffer zu Proben;
- Hinzufügen von 50 µl Proteinase K-Lösung;
- Übertragen der Probengefäße auf einen Heizschüttler und Inkubation für 2 Stunden bei 65°C unter Schütteln
- Hinzufügen von 50 µl Magnetpartikelsuspension;
- Inkubation 10 min. bei 65°C unter Schütteln;
- Inkubation 5 min. ohne Schütteln;
- Transfer der Probenröhrchen vom Heizschüttler zu einem Magneten
- Magnetisieren der Proben 3 min.;
- Transfer des Probenlysats zu einer Deep-well Probenplatte (DWP);
- Hinzufügen von 600 µl Bindungspuffer (Chaotrop);
- Hinzufügen von 50 µl Magnetpartikelsuspension in die DWP;
- Inkubation für 10 min. bei Raumtemperatur unter Schütteln;
- Transfer der DWP zu Magneten;
- Inkubation 5 min. bei Raumtemperatur im Magnetfeld;
- Absaugen und Verwerfen des Überstands;
- Transfer der DWP von Magnet zum Heizschüttler;
- Hinzufügen von 850 µl Waschpuffer 1;
- 10 sek. Schütteln bei Raumtemperatur;
- Transfer der DWP zum Magneten;
- Magnetisierung 2 min. bei Raumtemperatur;
- Absaugen und Verwerfen des Überstands;
- Transfer der DWP vom Magneten zum Heizschüttler;
- Hinzufügen von 450 µl Waschpuffer 2;
- 10 sek. Schütteln bei Raumtemperatur;
- Transfer der DWP zum Magneten;
- Magnetisierung 2 min. bei Raumtemperatur;
- Absaugen und Verwerfen des Überstands;
- Transfer der DWP von Magnet zum Heizschüttler;
- Hinzufügen von 850 µl Waschpuffer 3;
- 10 sek. Schütteln bei Raumtemperatur;
- Transfer der DWP zum Magneten;
- Magnetisierung 2 min. bei Raumtemperatur;
- Absaugen und Verwerfen des Überstands;
- Hinzufügen von 100 µl Elutionspuffer;
- Transer der DWP von Magnet zum Heizschüttler;
- Inkubation 10 min. bei 70°C unter Schütteln;
- Tranfer der DWP zum Magneten;
- Hinzufügen von 12 µl DNase-Mix (10 µl 10x DNase-Puffer; 2 µl DNase 1);
- Transfer der DWP von Magnet zum Heizschüttler (heruntergekühlt auf 37°C);
- Inkubation 30 min. bei 37°C ohne Schütteln;
- Transfer der DWP zu Magnet;
- Übertragen der DNase-verdauten Proben auf 1,5 ml Probengefäße;
- Ende Aufreinigungsprogramm
- Einfrieren der Proben und/ oder weitere Analyse der RNA Ausbeute.

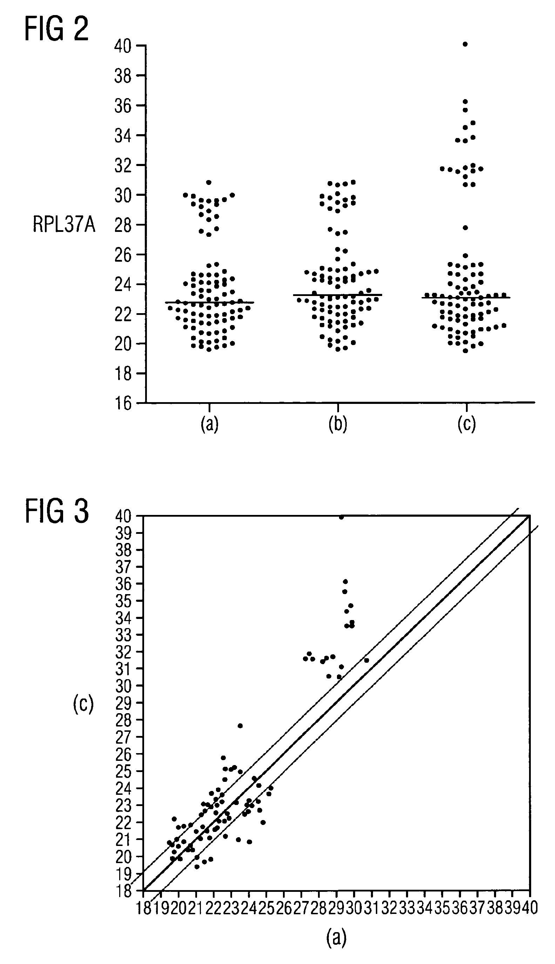

Fig. 2 zeigt einen Vergleich der Ausbeute (je niedriger der CT Wert für RPL37A desto höher) zwischen einem ersten manuell durchgeführten erfindungsgemäßen Verfahren mit Entfernung von Debris durch Zentrifugation (a), einem zweiten automatisiert durchgeführten erfindungsgemäßen Verfahren mit Entfernung von Debris durch magnetische Partikel (b), und einem manuellen Verfahren gemäß dem Stand der Technik (c) (Qiagen RNeasy FFPE Kit, Qiagen GmbH, Hilden, Deutschland, Verfahren gemäß Herstellerangaben).

Fig 3 zeigt eine Korrelation der Ausbeute (je niedriger der CT Wert für RPL37A desto höher die Expression des Gens) zwischen einem Verfahren gemäß dem Stand der Technik(Qiagen RNeasy FFPE Kit, (c)) und einem manuell durchgeführten erfindungsgemäßen Verfahren mit Entfernung von Debris durch Zentrifugation (a).

### Beispiel 5: Vergleich der Ausbeuten nach Aufreinigung mit den in Beispiel 1 und 2 beschriebenen Verfahren

Dieses Beispiel veranschaulicht den Effekt des Entfernens von zellulärem Debris durch einen zusätzlichen Reinigungsschritt mit magnetischen Partikeln (Beispiel 2) oder durch Zentrifugation (Beispiel 1). Zusätzlich wurden Proben aufgereinigt, bei denen das zelluläre Debris vor der Zugabe von chaotropem Puffer nicht entfernt wurde. Die anschließenden Schritte wurden bei allen Proben auf identische Weise, wie in Beispiel 1 beschrieben, durchgeführt.

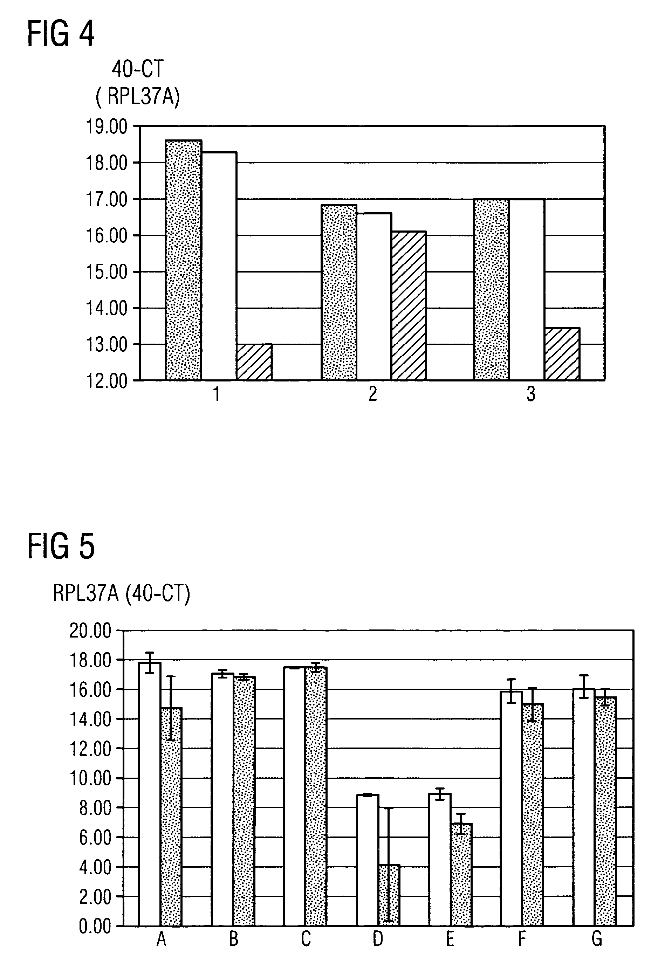

Fig. 4 zeigt einen Vergleich der Ausbeute einer manuellen Aufreinigung nach erfindungsgemäßen Verfahren mit Zentrifugation gemäß Beispiel 1 (gepunktete Säulen), mit zusätzlichem Beadbindungsschritt für Gewebe Debris gemäß Beispiel 2 (weiße Säulen) sowie nach einem manuellen Verfahren gemäß Stand der Technik ohne Entfernung von Zelldebris (schraffierte Säulen).

In Figur 4 sind Ergebnisse für 3 unterschiedliche Gewebeproben ("1", "2" und "3") gezeigt. Es zeigt sich, dass die Aufreinigung mit der erfindungsgemäßen Entfernung von zellulärem Debris zu einer signifikant höheren Ausbeute im Vergleich zu Proben führt, bei denen das zelluläre Debris nicht entfernt wurde. Der Ausbeutevergleich erfolgte über quantitative PCR des housekeeping-Gens RPL37A, wobei als Angabe für die Transkriptmenge 40-CT (CT = cycle threshold, d.h. die Anzahl von Amplifikationszyklen, bei welchen die Messschwelle des Systems überschritten wird). Eine Differenz von 3 bis 5 CT-Werten von RPL37A entspricht einem Ausbeuteverlust von Faktor 8 bis 32 der Gesamt-RNA. Dies führt zu der Schlussfolgerung, dass einerseits unlysiertes Gewebe bzw. zelluläres Debris die effiziente und quantitative Aufreinigung von Nukleinsäuren, insbesondere von RNA, beeinträchtigt und vermutlich die Bindung von Nukleinsäuren an silikabeschichtete magnetische Partikel unter chaotropen Bedingungen stört. Des Weiteren wird gezeigt, dass sich der anfängliche Zentrifugationsschritt unter nicht-chaotropen Bedingungen zur Entfernung von zellulärem Debris durch einen zusätzlichen Aufreinigungsschritt mit silikabeschichteten magnetischen Partikeln unter nicht-chaotropen Bedingungen substituieren lässt. Dies hat den erheblichen Vorteil, da das Verfahren damit sehr viel einfacher automatisierbar ist, da keine weiteren Zentrifugationsschritte notwendig sind.

### Beispiel 6: Vergleich der Ausbeute aus der automatisierten Aufreinigung mit und ohne Bindung von zellulärem Debris mittels silikabeschichteten Magnetpartikeln gemäß der beschriebenen Beispiele 3 und 4

Dieses Experiment veranschaulicht den Effekt des Entfernens von zellulärem Debris durch einen zusätzlichen Aufreinigungsschritt mit silikabeschichteten magnetischen Partikeln (entsprechend Beispiel 4) im Vergleich zu der automatisierten Aufreinigung ohne zusätzliche Aufreinigung von zellulärem Debris mit magnetischen Partikeln unter nicht-chaotropen Bedingungen (entsprechend Beispiel 3). Für jedes der beiden Verfahren wurden 3 aufeinanderfolgende Schnitte von 7 unterschiedlichen Gewebeproben ("A" bis "G") verwendet und bezüglich der Expression von RPL37A analysiert.

Fig. 5 zeigt die RNA Ausbeute im Vergleich des automatisierten erfindungsgemäßen Verfahrens (entsprechend Beispiel 4; weiße Säulen) mit einem automatisierten Verfahren aus dem Stand der Technik (entsprechend Beispiel 3; gepunktete Säulen) für 7 unterschiedliche Proben (A-G). Es zeigt sich, dass der zusätzliche magnetische Aufreinigungsschritt in 5 von 7 Fällen zu besseren Ausbeuten führte und dieser Schritt auch zwischen den 3 aufeinanderfolgenden Schnitten aus einer Gewebeprobe zu besser reproduzierbaren Ergebnissen führte (geringere Varianz innerhalb von unterschiedlichen RNA Präparationen aus dem gleichen Paraffinblock).

### Beispiel 7: Auswirkung der Gewebebindung auf die Effizienz und interferenzfreien Pipettierbarkeit der automatisierten Aufreinigung

Das VERSANT kPCR System der Firma Siemens, welches einen Pipettierrobotor der Firma Hamilton beinhaltet, ermöglicht die Kontrolle aller Aspirations- und Dispensionsschritte für jeden einzelnen Pipettierschritt. Die Flüssigkeitsbewegungen werden über Drucksensoren, die in den einzelnen Pipettierkanälen enthalten sind, aufgezeichnet. Diese Änderungen der Druckverhältnisse während jedes Pipettierschrittes werden über die Zeit aufgezeichnet (= TADM, Total Aspiration and Dispense Monitoring). Für jeden Pipettierschritt können bestimmte Toleranzbereiche für die Änderung des Druckverhältnisse definiert werden. Sobald das TADM-Profil außerhalb des definierten Bereiches liegt, kann direkt vermerkt werden, dass der Pipettierschritt bei einer Probe nicht ordnungsgemäß durchgeführt wurde, sei es herbeigeführt durch Verstopfung der Spitzen, Fehlen von Flüssigkeit, Schaumbildung in der Flüssigkeit oder andere negative Einflüsse. Die Probe kann daraufhin für weitere Analysen markiert bzw. unter Umständen auch von einer weiteren Analyse ausgeschlossen werden. In der klinischen Diagnostik würde diese Information in vielen Fällen zu einem Reflex- bzw. Wiederholungstest führen, entweder auf dem gleichen System oder einer alternativen Methode.

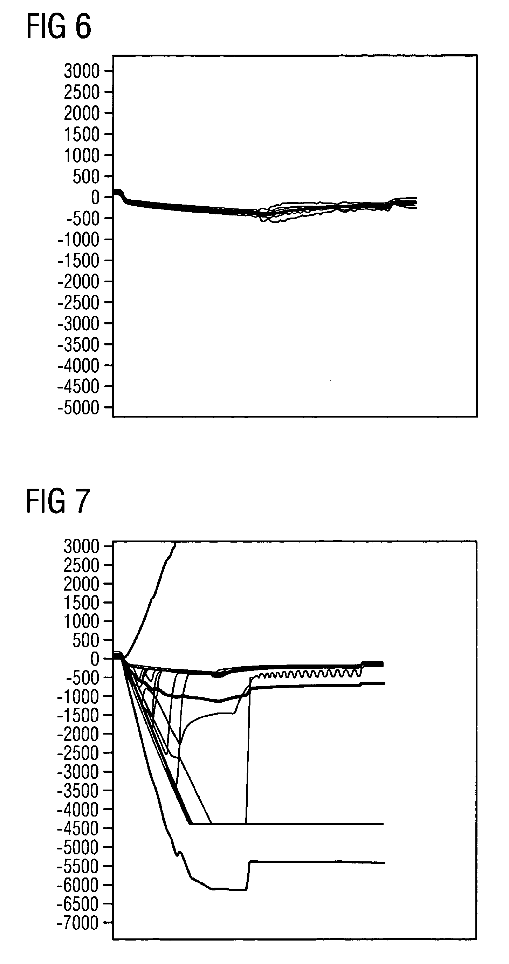

Abbildungen 6 und 7 zeigen TADM-Profile für die Aspiration der Lysate von fixierten Paraffinschnitten für den Transfer in ein neues Gefäß zur Nukleinsäurebindung unter chaotropen Bedingungen. Diese TADM Profile entsprechen dem Druckverlauf in der Pipette für diesen Pipettierschritt von jeder einzelnen Probe. Je näher die einzelnen Kurven beieinander liegen, um so konsistenter, d.h. kontrollierbarer ist der Druckverlauf bei Anwendung eines bestimmten Verfahrens. In diesem Beispiel handelt es sich zum einen um Proben (entsprechend Beispiel 4), bei denen zuvor magnetische Partikel zur Entfernung von Zelldebris zugegeben wurden (Abb. 6) und zum anderen um Proben (entsprechend Beispiel 3), bei denen keine Zugabe von Partikeln bzw. Abtrennung von Debris erfolgte (Abb. 7). Im Gegensatz zu Abbildung 6 zeigt die Abbildung 7 ein sehr viel ungleichmäßigeres TADM-Profil. Dies ist insbesondere dadurch zu erklären, dass bei diesen Proben noch Zelldebris im Lysat enthalten war, das zu Verstopfung der Spitzen und dadurch zu sehr hoher Druckentstehung führen kann. In manchen Fällen kann es bei automatisierten Verfahren ohne vorherige Entfernung von zellulärem Debris sogar zu einem Abbruch eines Verfahrensablaufs kommen. Dies kann durch das erfindungsgemäße Verfahren effektiv verhindert werden. Wenn beim erfindungsgemäßen Verfahren zur Entfernung von Zellulärem Debris Magnetpartikel verwendet werden, hat dies den zusätzlichen Vorteil, dass bei dem Schritt der Entfernung von Debris die gleiche Hardware verwendet werden kann, die auch zur Isolierung von Nukleinsäuren mit Magnetpartikeln unter chaotropen Bedingungen verwendet wird.

Dieses Beispiel zeigt, dass durch die Entfernung von Zelldebris die Pipettierbarkeit (z.B. Aspiration der Lyseflüssigkeit) und Effizienz der automatisierten Aufreinigung verbessert wird, da es seltener zum Ausschluß einer Probe aufgrund eines schlechten TADM Profils kommt. Dadurch wird in der klinischen Diagnostik die Anzahl an Reflextests (erneute Durchführung des Tests, der zu einem Ergebnis führt) deutlich erniedrigt, was mit einer Kostenreduktion einhergeht.

### Beispiel 8: Aufreinigung von Nukleinsäuren aus Blutproben

Das erfindungsgemäße Verfahren ist insbesondere auch zur Aufreinigung von Nukleinsäuren aus Blutproben geeignet, weil es eine verbesserte Entfernung von Hämoglobin oder Erythrozytenfragmenten aus dem Blut ermöglicht. Erfindungsgemäß werden unter nicht-chaotropen Bedingungen zunächst effizient Erythrozyten, Erythrozytenfragmente und auch freigesetztes Hämoglobin aus der Probe entfernt, welche spätere Verfahrensschritte stören können.

Gemäß einer ersten Variante wird zu einer Blutprobe (z.B. 100 µl EDTA-Gesamtblut) 400 µl Lysepuffer (z.B. 10 mMol TRIS-HCL, 0,1 mMol EDTA, 2% SDS, pH 8,0) gegeben. Anschließend erfolgt Zugabe von 50 µl Magnetpartikelsuspension (z.B. unbeschichtete oder silikabeschichtete Magnetpartikel) zu der Probe, Inkubation 10 min bei Raumtemperatur und Abtrennen der Partikel durch Anlegen eines Magnetfeldes. Aus der abgenommenen Probe können anschließend, wie oben beschrieben, unter chaotropen Bedingungen Nukleinsäuren isoliert werden.

Gemäß einer zweiten Variante wird die Blutprobe in einem hypotonen Lysepuffer (z.B. 25 mM TRIS HCl, pH 7,5, 10 mM KCl. 5 mM MgCl₂ )aufgenommen, kurz inkubiert um die Erythrozyten zu lysieren und anschließend erfolgt die Zugabe Magnetpartikelsuspension (z.B. unbeschichtete oder silikabeschichtete Magnetpartikel) zu der Probe, Inkubation von 10 min bei Raumtemperatur und abtrennen der Partikel mitsamt der Erythorzytenfragmente und des Hämoglobins durch Anlegen eines Magnetfeldes. Anschließend erfolgt die Lyse der Leukozyten unter chaotropen Bedingungen, d.h. Freisetzung der Nukleinsäuren sowie Bindung dieser an frisch zugegebene Silika beschichtete Magnetpartikel. Proteinase K kann optional zuvor oder zeitgleich mit dem chaotropen Reagenz hinzugeführt werden.

### Beispiel 9: Herstellung von silika-beschichteten magnetischen Partikeln:

Die Herstellung von silika-beschichteten magnetischen Partikeln kann z.B. durch silika-Beschichtung von Magnetitpartikeln erfolgen. Bei den eingesetzten Magnetiten handelt es sich vorzugsweise um hydrophile, kommerziell erhältliche Eisenoxide (Fe₃O₄), die bevorzugt in enger Korngrössenverteilung und kugelförmiger Morphologie verfügbar sind. Magnetitpartikel sind kommerziell erhältlich, derartige Produkte werden beispielsweise von der Fa. Bayer AG unter dem Produktnamen BAYOXIDE E hergestellt. Geeignete Typen sind unter der Bezeichnung BAYOXIDE E8706, E8707, E8709 und E8710 erhältlich. Ähnliche Produkte werden auch von BASF unter dem Namen "Magnetic Pigment 340" bzw. "345" vertrieben. Obwohl mit allen genannten Produkten gute Ergebnisse erzielt werden können, wird vorzugsweise der Typ BAYOXIDE E 8707 oder E 8706 verwendet. Dieses magnetische Pigment besitzt eine kugelförmige Morphologie bei einem mittleren Teilchendurchmesser von 0,2 um und enger Korngrössenverteilung (ca. 0,1 bis 0,7 um). Als Ausgangsmaterialien zum Einführen von Silikatgruppen können sowohl Alkali- silikate (Natron- oder Kali-Wassergläser) als auch Kieselsole eingesetzt werden. Geeignete Wassergläser, die üblicherweise sehr hohe pH Werte (13-14) besitzen, werden von verschiedenen Firmen, beispielsweise Merck oder Cognis angeboten. Das zu beschichtende Material, beispielsweise Bayoxide E 8707, kann unter Rühren in eine verdünnte beispielsweise 1 % ige Wasserglaslösung gegeben werden. Nach einer Inkubation von ca. 30 Minuten wird abfiltriert, mit Wasser gewaschen und getrocknet. Gemäß einem exemplarischen Protokoll werden in 1000 ml einer wässrigen 0,25 % igen Wasserglaslösung (HK30; Cognis) unter Rühren 50 g Bayoxide E 8707 gegeben, anschließend wird noch 30 Min. bei RT nachgerührt Die Partikel werden abfiltriert, 5-mal mit Wasser und einmal mit Ethanol gewaschen und anschließend 5 Std. bei 80° C getrocknet.

## Patentansprüche

1. Ein Verfahren zum Aufreinigen von Nukleinsäuren aus einer biologischen Probe, aufweisend die folgenden Schritte:
a) Aufnehmen der Probe in einer wässrigen Lösung;
b) Lysieren der Probe;
c) Abtrennen von zellulärem Debris; und
d) Isolieren der Nukleinsäuren aus der Lösung;
wobei Schritte (a) bis (c) unter nicht-chaotropen Bedingungen stattfinden, wobei Schritt (c) aufweist: Suspendieren von magnetischen Partikeln in der Lösung und Anwenden eines magnetischen Feldes zum Zurückhalten der magnetischen Partikel und Abtrennen der magnetischen Partikel von der Lösung.

2. Verfahren nach Anspruch 1, wobei die Magnetpartikel eine siliziumhaltige Beschichtung, insbesondere eine Siliziumdioxid enthaltende Beschichtung, aufweisen.

3. Verfahren nach einem der vorangehenden Ansprüche, wobei die Probe eine Blutprobe ist.

4. Verfahren nach einem der Ansprüche 1 oder 2, wobei die biologische Probe eine in Paraffin eingebettete Probe und/oder eine fixierte Probe ist.

5. Verfahren nach einem der vorangehenden Ansprüche, wobei die Lösung vor dem Schritt (d) auf mindestens 50°C, bevorzugt mindestens 60°C, stärker bevorzugt 60 - 95°C, erhitzt wird.

6. Verfahren nach Anspruch 4, wobei die Probe nach dem Erhitzen vor dem Schritt (d) auf unterhalb 50°C abgekühlt wird.

7. Verfahren nach einem der vorangehenden Ansprüche, wobei die Probe in mindestens einem der Schritte a), b) und c) mit einer hydrophoben Matrix in Kontakt gebracht wird.

8. Verfahren nach einem der Ansprüche 1 bis 5, wobei die Lösung in Schritt (c) durch Absaugen von den Magnetpartikeln abgetrennt wird.

9. Verfahren nach einem der vorangehenden Ansprüche, wobei Schritt (d) das Hinzufügen einer chaotropen Verbindung zu der Probe umfasst.

10. Verfahren nach Anspruch 9, wobei Schritt (d) ferner das Hinzufügen von unverbrauchten magnetischen Partikeln mit siliziumhaltiger Beschichtung zu der Lösung umfasst.

11. Verwendung von Magnetpartikeln zur Entfernung von zellulärem Debris aus einer biologischen Probe in einer wässrigen Lösung unter nicht- chaotropen Bedingungen, aufweisend:
Suspendieren von magnetischen Partikeln in der Lösung und Anwenden eines magnetischen Feldes zum Zurückhalten der magnetischen Partikel und Abtrennen der magnetischen Partikel von der Lösung.

12. Verwendung nach Anspruch 11, wobei die magnetischen Partikel eine siliziumhaltige Beschichtung, insbesondere eine Siliziumdioxid enthaltende Beschichtung, aufweisen.

13. Verwendung von magnetischen Partikeln mit siliziumhaltiger Beschichtung nach Anspruch 11 zur Durchführung des Verfahrens gemäß einem der Ansprüche 1 bis 9.

## Claims

1. A method for purifying nucleic acids from a biological sample, comprising the steps of:
a) receiving the sample in an aqueous solution;
b) lysing the sample;
c) separating cellular debris; and
d) isolating the nucleic acids from the solution;
wherein steps (a) to take place through (c) under non-chaotropic conditions, wherein step (c) comprises: suspending magnetic particles in the solution and applying a magnetic field for retaining the magnetic particles and separating the magnetic particles from the solution.

2. Method according to claim 1, wherein the magnetic particles have a silicon-containing coating, in particular a silica-containing coating.

3. Method according to one of the preceding claims, wherein the sample is a blood sample.

4. Method according to one of claims 1 or 2, wherein the biological sample is a sample embedded in paraffin and/or a fixed specimen.

5. Method according to one of the preceding claims, wherein the solution prior to step (d) is heated to a temperature of at least 50 °C, preferably at least to 60 °C, more preferably to 60-95 °C.

6. Method according to claim 4, wherein the sample after heating before step (d) is cooled to below 50 °C.

7. Method according to one of the preceding claims, wherein the sample is brought in contact with a hydrophobic matrix at least in one of steps a), b) and c).

8. Method according to one of claims 1 to 5, wherein the solution in step (c) is separated from the magnetic particles by aspiration.

9. Method according to one of the preceding claims, wherein step (d) comprises adding a chaotropic compound to the sample.

10. Method of claim 9, wherein step (d) further comprises the addition of magnetic particles with silicon-unused coating to the solution.

11. The use of magnetic particles for the removal of cellular debris from a biological sample in an aqueous solution under non-chaotropic conditions, comprising: suspending magnetic particles in the solution and applying a magnetic field for retaining the magnetic particles and separating the magnetic particles from the solution.

12. Use according to claim 11, wherein the magnetic particles have a silicon-containing coating, in particular a silica-containing coating.

13. The use of magnetic particles with silicon-containing coating according to claim 11 for implementing the method according to any one of claims 1 to 9.

## Revendications

1. Procédé de purification d'acides nucléiques d'un échantillon biologique, présentant les étapes suivantes:
a) Absorption de l'échantillon dans une solution aqueuse;
b) Lyse de l'échantillon;
c) Séparation des débris cellulaires, et
d) Isolement des acides nucléiques hors de la solution;
dans lequel les étapes (a) à (c) ont lieu dans des conditions non-chaotropiques, l'étape (c) présentant:
la mise en suspension de particules magnétiques dans la solution et l'application d'un champ magnétique pour retenir les particules magnétiques et la séparation des particules magnétiques de la solution.

2. Procédé selon la revendication 1, dans lequel les particules magnétiques présentent un revêtement siliceux, en particulier un revêtement contenant du dioxyde de silicium.

3. Procédé selon l'une des revendications précédentes, dans lequel l'échantillon est un échantillon sanguin.

4. Procédé selon l'une des revendications 1 ou 2, dans lequel l'échantillon biologique est un échantillon noyé dans de la paraffine et/ou un échantillon fixé.

5. Procédé selon l'une des revendications précédentes, dans lequel la solution avant l'étape (d) est chauffée à au moins 50°C, de préférence à au moins 60°, de manière plus fortement préférée de 60 à 95°C.

6. Procédé selon la revendication 4, dans lequel l'échantillon est, après le chauffage avant l'étape (d), refroidi à moins de 50°C.

7. Procédé selon l'une des revendications précédentes, dans lequel l'échantillon est, dans au moins une des étapes a), b) et c), mis en contact avec une matrice hydrophobe.

8. Procédé selon l'une des revendications 1 à 5, dans lequel la solution est séparée à l'étape (c) par aspiration des particules magnétiques.

9. Procédé selon l'une des revendications précédentes, dans lequel l'étape (d) comprend l'addition d'un composé chaotropique à l'échantillon.

10. Procédé selon la revendication 9, dans lequel l'étape (d) comprend en outre l'addition à la solution de particules magnétiques non utilisées avec revêtement siliceux.

11. Utilisation de particules magnétiques pour l'élimination des débris cellulaires hors d'un échantillon biologique dans une solution aqueuse dans des conditions non-chaotropiques, présentant:
la mise en suspension de particules magnétiques dans la solution et l'application d'un champ magnétique pour retenir les particules magnétiques et la séparation des particules magnétiques de la solution.

12. Utilisation selon la revendication 11, dans laquelle les particules magnétiques présentent un revêtement siliceux, en particulier un revêtement contenant du dioxyde de silicium.

13. Utilisation de particules magnétiques avec revêtement siliceux selon la revendication 11 pour la mise en oeuvre du procédé selon l'une des revendications 1 à 9.
